# FASCICULE DE BREVET EUROPEEN

(11) **EP 3 541 459 B1**
(45) Date de publication et mention de la délivrance du brevet: **26.07.2023**
(21) Numéro de dépôt: 17794995.5
(22) Date de dépôt: 14.11.2017
(51) Int. Cl.: A61M 25/00, A61M 1/36

(54) **CANULE, SYSTÈME D'ASSISTANCE ECMO**
KANÜLE, ECMO-STÜTZSYSTEM
CANNULA, ECMO SUPPORT SYSTEM

(30) Priorité: 15.11.2016 FR 1661037
(43) Date de publication de la demande: 25.09.2019
(73) Titulaire: Assistance Publique Hôpitaux de Paris, 75004 Paris (FR)
(72) Inventeur: MORDANT, Pierre, 75011 Paris (FR)
(74) Mandataire: Cabinet Laurent & Charras
(86) Numéro de dépôt international: PCT/EP2017/079219
(87) Numéro de publication internationale: WO 2018/091474

(56) Documents cités:
- WO-A1-2011/161146
- WO-A1-2016/137212
- US-A- 4 114 618
- US-A- 5 171 218
- US-A1- 2012 259 273
- US-A1- 2016 121 079

## Description

### DOMAINE TECHNIQUE DE L'INVENTION

La présente invention concerne une canule pour l'injection d'un fluide dans un organe, un vaisseau sanguin artériel ou veineux, un vaisseau lymphatique, ou une structure bronchique. Plus particulièrement, le domaine de l'invention se rapporte aux canules pour les systèmes extra corporels permettant l'oxygénation d'un flux de sang prélevé et réintroduit dans un organe ou un vaisseau identique ou différent de l'organe ou du vaisseau de prélèvement. Plus particulièrement, le domaine de l'invention se rapporte aux canules pour les systèmes ECMO (Extra Corporeal Membrane Oxygénation) permettant l'oxygénation d'un flux de sang prélevé et réintroduit dans un organe ou un vaisseau. Enfin, l'invention se rapporte notamment aux dispositifs ECMO-VA (veino-artérielle) permettant d'acheminer un premier volume de sang oxygéné dans une artère dans le sens rétrograde et un second volume de sang oxygéné dans ladite artère dans le sens antérograde.

### ARRIERE PLAN TECHNOLOGIQUE DE L'INVENTION

Il existe aujourd'hui des canules associées à des dispositifs d'oxygénation par membrane extracorporelle, connue sous l'acronyme ECMO. Ces canules permettent d'extraire un volume de sang grâce à une pompe afin d'injecter un volume de sang traité extra corporellement pour être oxygéné. Il existe toutes sortes de canules selon les applications souhaitées qui peuvent être notamment des applications Veino-veineuse VV ou veino-artérielle VA ou encore veino-artério-veineuse VAV. Ces canules permettent une circulation du sang dans un circuit fermé grâce à une technique d'oxygénation d'un volume de sang pauvre en oxygène prélevé d'un corps.

L'ECMO-VA, désignant « Extra Corporeal Membrane Oxygénation - Veino Artérielle », est un traitement de référence du choc cardiogénique réfractaire, de l'arrêt cardiaque réfractaire, de la décompensation d'hypertension artérielle pulmonaire primitive ou secondaire. Elle est une technique privilégiée en cas de mauvaise tolérance hémodynamique ou respiratoire de la transplantation pulmonaire, de la transplantation cardiaque, et de toute chirurgie thoracique pulmonaire ou cardiovasculaire.

Une ECMO comprend un ensemble de composants permettant d'extraire un volume sanguin d'un organe ou d'un vaisseau, de l'oxygéner, de le décarboxyler, éventuellement de le chauffer, puis de le réinjecter oxygéné artificiellement dans un organe ou un vaisseau.

Elle est désignée comme une « ECMO-VA périphérique » lorsque l'injection de sang oxygéné est effectuée dans une artère périphérique et non dans la racine aortique ou dans l'artère pulmonaire (ECMO-VA centrale).

Le circuit d'ECMO-VA périphérique comprend généralement une canule d'admission qui est préférentiellement placée dans une veine de grand diamètre, une première tubulure héparinée, une pompe centrifuge, une membrane d'oxygénation et de décarboxylation, une seconde tubulure héparinée et une canule d'injection qui est elle aussi placée préférentiellement dans une artère de grand diamètre.

Un problème rencontré actuellement concerne la mise en place de la canule d'injection artérielle dans une artère périphérique du fait d'un risque important d'occlusion artérielle et donc d'ischémie de membre, tel qu'un bras ou une jambe. Typiquement, lorsque la canule d'injection est insérée dans l'artère axillaire ou dans l'artère fémorale, elle peut entrainer un risque de diminution du débit d'aval causé par la présence de la canule et par la création d'un flux artériel rétrograde. La notion d'«aval» et de « rétrograde » est définie par rapport au sens de circulation physiologique du sang.

La diminution du débit d'aval peut alors engendrer une altération du flux artériel de perfusion du membre et créer une ischémie de membre. Cette conséquence est d'autant plus marquée que l'hémodynamique est altérée et/ou que l'hématose est altérée et/ou que la collatéralité est limitée.

Si l'ischémie de membre iatrogène n'est pas traitée, elle peut aboutir à une rhabdomyolyse, une insuffisance rénale, une dialyse et le décès. Si elle est traitée trop tard, il peut être nécessaire de réaliser des aponévrotomies de décharge, et possiblement une amputation de membre.

Aujourd'hui, afin de pallier aux conséquences d'une ischémie de membre sous ECMO-VA périphérique, une seconde canule dite de reperfusion peut être branchée en Y sur la ligne artérielle de l'ECMO. Cette seconde canule est implantée dans le sens antérograde, en aval de la canule principale qui est généralement implantée dans le sens rétrograde.

Cependant cette technique utilisant deux canules de diamètres différents, et devant être implantées en deux endroits différents de l'artère, dans deux sens différents, comporte des inconvénients.

Tout d'abord, c'est une procédure longue et nécessite parfois différents soignants, et des techniques d'imagerie complémentaires, artériographie sous radioscopie ou échographie-doppler vasculaire. Par ailleurs, elle complexifie la mise en place de l'ECMO dans un contexte souvent d'urgence, car il y a deux points de ponctions dans l'artère. En effet, les deux canules doivent être implantées dans des sens différents et doivent être manipulées avec précaution. En outre, elles possèdent des calibres différents de 20G à 6F pour la canule de reperfusion et de 15F à 18F pour la canule d'injection. Un autre inconvénient est que la canule de reperfusion peut causer des complications supplémentaires chez le patient telles que des embols distaux, une dissection de l'artère, une thrombose de l'artère, ou un hématome au point de ponction.

Il est connu aussi du document US2016121079, une canule double direction pour perfuser le sang dans deux directions. La canule a une ouverture distale en communication fluidique avec une première lumière de canule et a une ouverture secondaire à proximité de l'ouverture distale et une extension tubulaire mobile pouvant s'étendre à partir de la canule via l'ouverture secondaire.

### RESUME DE L'INVENTION

L'invention, définie à la revendication 1 et définie plus avant aux revendications dépendantes, permet de résoudre les inconvénients précités.

Un objet de l'invention concerne une canule pour la circulation d'un fluide dans une artère, caractérisée en ce qu'elle comprend :
▪ Une lumière principale acheminant un volume de fluide vers une première extrémité distale ;
▪ Une lumière accessoire comportant au moins une portion interne (29) agencée à l'intérieur de la lumière principale et s'étendant en partie parallèlement à cette dernière sur une portion de la longueur de la lumière principale, ladite lumière accessoire comportant :
   o une extrémité proximale située en aval de l'extrémité proximale de la lumière principale de sorte à capter une fraction du débit entrant du fluide dans la lumière principale ;
   o une portion coudée modifiant la direction d'écoulement du débit de fluide captée par la lumière accessoire vis-à-vis de la direction d'écoulement du fluide débouchant de la première extrémité;
   o une seconde extrémité distale située en amont de la première extrémité distale de la lumière principale, débouchant sur une ouverture latérale de la canule de sorte à diriger ladite fraction captée du liquide dans la direction modifiée d'écoulement, la seconde extrémité distale de la lumière accessoire correspondant à l'ouverture latérale.

Un avantage est de limiter l'effet d'occlusion artérielle et donc d'ischémie du membre. Cette solution élimine la mise en place d'une canule de reperfusion difficile à mettre en place. En outre le fait d'avoir la seconde extrémité distale correspondant à une ouverture latérale dans une paroi formant la lumière principale de la canule permet de diminuer le risque important d'occlusion artérielle et donc d'ischémie. En effet, le fait d'insérer une lumière accessoire dans artère diminue la section passante du sang et peut provoquer un thrombus ou une occlusion. Le fait que la portion de la lumière accessoire ne soit pas à l'extérieur de la canule évite de diminuer la section de l'artère, et évite donc un risque de thrombus ou d'occlusion.

Selon un mode de réalisation, la portion coudée est conçue de sorte à orienter le flux capté par la lumière accessoire dans une direction sensiblement opposée au flux s'écoulant vers l'extrémité de la lumière principale.

Selon un mode de réalisation, la lumière principale comporte un diamètre de 15F à 20F, soit de 5 à 7 mm et que la lumière accessoire compote un diamètre de 20G à 6F, soit de 0,9 à 2mm. Un avantage est de permettre de générer un débit antérograde en amont de l'injection dans le sens rétrograde de la lumière principale de la canule pour limiter les risques d'ischémie dans la partie amont du membre.

Selon un mode de réalisation, la lumière accessoire a une section circulaire ou elliptique.

Selon un mode de réalisation, l'extrémité proximale et/ou distale de la lumière accessoire est biseautée de sorte que la section de l'embouchure proximale et/ou distale de la lumière accessoire s'inscrive dans un plan non orthogonal à l'axe de l'une des deux lumières. Un avantage est de « laminariser » le flux sanguin en entrée de la lumière accessoire et limiter les turbulences.

Selon un mode de réalisation, la lumière accessoire comporte une partie longitudinale fixée le long d'une portion de la surface intérieure de la lumière principale. Un avantage est de permettre de maintenir la lumière accessoire en place.

Selon un mode de réalisation, la lumière accessoire est formée d'une unique portion interne et comprise dans le volume de la lumière principale.

Selon un mode de réalisation, la lumière accessoire comprend en outre une portion externe à la lumière principale agencée en amont de la portion interne, la dite portion externe présentant un robinet de purge et étant destinée à être une portion extracorporelle de la canule.

Selon un mode de réalisation, la portion externe de la lumière accessoire comporte une première jonction agencée au contact de la surface latérale de la lumière principale et une seconde jonction agencée au contact de la surface latérale de la lumière principale. La portion externe permet d'acheminer une fraction du liquide qui est acheminé vers la première extrémité distale de la lumière principale extérieurement à ladite lumière principale entre les deux jonctions. Cette fraction qui est acheminé extérieurement à la lumière principale peut être purgée grâce à la présence au robinet de purge.

Il est ici également décrit une canule pour la circulation d'un fluide dans une artère, la canule , qui n'est pas revendiquée et ne forme donc pas partie de l'invention, comprend: :
- une lumière principale comprenant une première extrémité distale et une première extrémité proximale, la lumière principale acheminant un volume de fluide suivant une longueur entre la première extrémité proximale et la première extrémité distale ;
- une lumière accessoire comportant
   - au moins une portion interne agencée à l'intérieur de la lumière principale et s'étendant en partie parallèlement à cette dernière sur une portion de la longueur de la lumière principale,
   - une extrémité proximale située en aval de l'extrémité proximale de la lumière principale de sorte à capter une fraction du débit entrant du fluide dans la lumière principale ;
   - une portion coudée modifiant la direction d'écoulement du débit de fluide captée par la lumière accessoire vis-à-vis de la direction d'écoulement du fluide débouchant de la première extrémité ;
   - une seconde extrémité distale située en amont de la première extrémité distale de la lumière principale, débouchant en dehors de la lumière principale,
caractérisé en ce que la lumière accessoire comprend en outre une portion externe à la lumière principale agencée en amont de la portion interne, la dite portion externe de la lumière accessoire présentant une partie laissant passer la lumière à l'intérieur de la portion externe. cette portion externe de la lumière accessoire est destinée à être une portion extracorporelle de la canule. Ainsi, lors de la mise en place de la canule, l'utilisateur peut voir si la seconde extrémité distale est dans l'artère, du fait du sang remontant dans cette lumière et donc passant au niveau de la portion externe laissant passer la lumière.

Selon un mode de réalisation, la partie externe de la lumière accessoire laissant passer la lumière est sur toute la longueur de la portion externe.

Selon un mode de réalisation, la partie externe de la lumière accessoire est translucide ou transparente.

Le fait d'être transparent permet en outre d'aider l'opérateur à estimer le débit visuellement du fluide.

Selon un mode de réalisation, la portion externe de la lumière accessoire comprend un robinet de purge.

Selon un exemple de ce mode de réalisation, le robinet comprend au moins un état fermé permettant au fluide de passer de l'extrémité distale de la lumière accessoire à l'extrémité proximale de la lumière accessoire (dans un sens ou dans l'autre) et un état purge empêchant le fluide de passer de l'extrémité distale de la lumière accessoire à l'extrémité proximale de la lumière accessoire. Ainsi, le fait que la partie externe soit translucide ou transparent permet à l'opérateur de voir le fluide que ce soit à l'état fermé ou à l'état ouvert.

Le robinet comprend dans cet exemple une sortie purge. Cette sortie purge est adaptée pour être raccordée à un tuyau.

Selon une particularité, à l'état purge, le robinet comprend une position antérograde permettant au fluide de passer l'extrémité proximale vers la sortie purge. Ainsi, l'opérateur peut vérifier le bon écoulement du sang depuis l'entrée de la canule connectée au circuit d'ECMO vers la lumière accessoire.

Selon une particularité, à l'état purge, le robinet peut avoir une position rétrograde permettant au fluide de passer l'extrémité distale vers la sortie purge. Ainsi, l'opérateur peut vérifier le bon écoulement du sang depuis la sortie de la canule positionnée dans l'artère vers la lumière accessoire. Par l'évaluation de ce reflux, l'opérateur peut vérifier la bonne position intra-artérielle de l'extrémité distale de la lumière accessoire.

Selon une particularité, à l'état fermé, le robinet comprend une sortie permettant au fluide de passer de l'extrémité proximale à l'extrémité distale de la lumière accessoire.

Selon un mode de réalisation, la lumière accessoire a son extrémité proximale à l'intérieure de la lumière principale et en ce que son extrémité proximale en aval de la première extrémité proximale, est biseautée de sorte que la section de l'embouchure proximale de la lumière accessoire s'inscrive dans un plan non orthogonal à l'axe de l'une des deux lumières. Un avantage est de « laminariser » le flux sanguin en entrée de la lumière accessoire et limiter les turbulences.

Selon un mode de réalisation, la seconde extrémité distale est agencée de sorte à diriger ladite fraction captée du liquide dans la direction modifiée d'écoulement ;

Selon un exemple de ce mode de réalisation, la seconde extrémité distale est une ouverture latérale dans une paroi formant la lumière principale de la canule.

Selon un autre exemple de ce mode de réalisation, la seconde extrémité distale comprend une portion située en dehors de la paroi formant la lumière principale. Par exemple, la seconde extrémité distale est rétractable à l'intérieure de la lumière principale pour l'insertion et le retrait de la canule dans l'artère et est en fonctionnement en dehors de la lumière principale.

Les différents modes de réalisations peuvent se combiner.

D'autre caractéristiques de la canule décrite précédemment peuvent aussi être présent dans cette canule.

Il est ici également décrit une canule, qui n'est pas revendiquée et ne forme donc pas partie de l'invention, la canule comprend au moins :
- une lumière principale comprenant une première extrémité distale et une première extrémité proximale, la lumière principale acheminant un volume de fluide suivant une longueur entre la proximité distale et la première extrémité distale ;
- une lumière accessoire comportant
   - au moins une portion interne agencée à l'intérieur de la lumière principale et s'étendant en partie parallèlement à cette dernière sur une portion de la longueur de la lumière principale,
   - une extrémité proximale située en aval de l'extrémité proximale de la lumière principale de sorte à capter une fraction du débit entrant du fluide dans la lumière principale ;
   - une portion coudée modifiant la direction d'écoulement du débit de fluide captée par la lumière accessoire vis-à-vis de la direction d'écoulement du fluide débouchant de la première extrémité ;
   - une seconde extrémité distale située en amont de la première extrémité distale de la lumière principale, débouchant en dehors de la lumière principale,
caractérisée en ce que la canule comprend en outre une valve anti-reflux entre l'extrémité distale de la lumière principale et l'extrémité proximale de la lumière accessoire.

Cela permet de diminuer, voir supprimer, lors de l'installation de la canule que du sang sort de la canule par l'extrémité proximale de la lumière principale.

La valve anti-reflux peut avoir une ouverture ou une fente permettant d'y insérer un introducteur. L'introducteur traverse donc la valve anti reflux. La valve anti reflux comprend une membrane ayant l'ouverture ou la fente agencée de manière que les parois de la membrane ferme l'ouverture ou la fente lorsque du fluide pousse la membrane vers l'extrémité proximale de la lumière principale.

La membrane est agencée pour laisser le fluide circuler dans le sens de l'extrémité proximale de la lumière principale vers l'extrémité distale de la lumière principale. Ainsi cela permet que lors du retrait d'un introducteur de la canule, de réduire le sang sortant de la canule.

En effet, il est connu d'utiliser un introducteur plus rigide que la canule et ayant son extrémité distale plus aiguisé pour permettre de moins abimer l'artère lors de l'insertion de la canule dans l'artère.

La lumière accessoire de la canule peut aussi avoir la portion externe à la lumière principale ou/et l'extrémité distale correspondant à l'ouverture latérale dans une paroi de la lumière principale.

La canule peut aussi avoir les ou la caractéristique d'une ou des autres modes de réalisations décrits précédemment pour les canules précédentes.

Un autre objet non-revendiqué et ne formant donc pas partie de l'invention concerne un ensemble canule introducteur caractérisé en ce qu'il comporte une des trois canules décrit précédemment et un introducteur ayant une portion traversant la lumière principale.

Un autre objet de l'invention concerne un système d'injection d'un fluide dans une artère, caractérisé en ce qu'il comporte :
▪ la canule de l'invention précédemment décrite,
▪ une pompe agencée pour assurer le pompage d'un débit de fluide prédéterminé provenant d'une canule de prélèvement, coopérant avec une entrée de la pompe via un tube d'interface ;
▪ un oxygénateur agencé pour introduire une proportion d'oxygène dans un fluide, une entrée de l'oxygénateur coopérant avec une sortie de la pompe via un second tube d'interface;
▪ un échangeur de chaleur agencé pour injecter un fluide à une température fixée dans la canule par l'intermédiaire d'un troisième tube d'interface, ledit échangeur prélevant le fluide de l'oxygénateur par l'intermédiaire d'un troisième tube.

Selon un mode de réalisation, au moins un tube est recouvert d'une couche d'héparine.

Selon un mode de réalisation, la pompe est agencée et configurée pour établir un débit de sang pulsé.

Un autre objet non-revendiqué et ne formant pas partie de l'invention concerne un système d 'injection d'un fluide dans une artère comprenant l'une des canules non-revendiquées et ne formant donc pas partie de l'invention ainsi qu'une pompe, un oxygénateur et un échangeur de chaleur tels que décrits pour le système d'injection d'un fluide précédent.

### BREVE DESCRIPTION DES FIGURES

D'autres caractéristiques et avantages de l'invention ressortiront à la lecture de la description détaillée qui suit, en référence aux figures annexées, qui illustrent :
- La figure 1 : un système ECMO comportant une canule d'injection de l'invention ;
- La figure 2 : une vue en perspective d'un mode de réalisation d'une canule de l'invention;
- La figure 3 : un mode de réalisation représentant une portion d'une canule de l'invention comportant une lumière principale et une lumière accessoire ;
- Les figures 4A à 4D : différentes variantes de réalisation du maintien de la lumière accessoire à l'intérieur de la lumière principale ;
- La figure 5 : un exemple de réalisation d'une jonction d'une canule accessoire débouchant en périphérie de la canule principale, ladite jonction étant agencée dans le prolongement d'un coupe formé par la lumière accessoire ;
- La figure 6 : un mode de réalisation dans lequel la lumière accessoire comporte une portion externe à la lumière principale comportant un robinet de purge ;
- La figure 7 différents modes de fonctionnement de purge pouvant être réalisés grâce au robinet.

### DESCRIPTION DETAILLEE DE MODES DE REALISATION DE L'INVENTION

Dans la suite de la description, on nomme une « direction d'écoulement antérograde » d'un fluide : la direction d'écoulement physiologique du sang dans un vaisseau ou un organe. On nomme une « direction d'écoulement rétrograde » d'un fluide la direction inverse d'écoulement du sang dans un vaisseau ou un organe. Le référentiel est donc pris par rapport à la physiologie du corps humain ou animal. Par extension de langage et pour la clarté de l'exposé du mode de réalisation, on nommera la direction d'écoulement de la lumière principale LP : la « direction rétrograde » et la direction d'écoulement de la lumière accessoire LAc : la « direction antérograde », chacune étant considérée en sortie de la canule 1 lorsque l'on évoque leur direction.

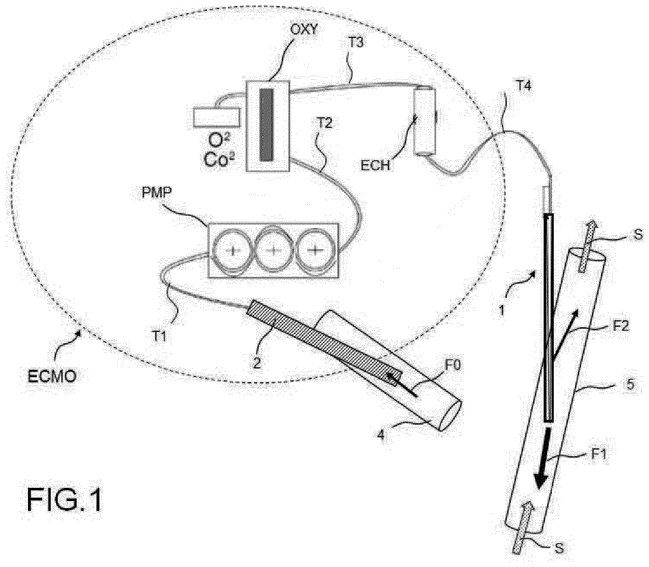

La figure 1 représente un système ECMO comportant différents composants permettant d'oxygéner un volume de sang prélevé et de le réinjecter dans un vaisseau d'un patient, telle qu'une artère.

Une pompe PMP assure deux rôles : le pompage du sang pauvre en oxygène et riche en dioxyde de carbone d'une veine 4 provenant de la canule d'admission 2 ainsi que l'injection du sang oxygéné et décarboxylé dans une canule d'injection 1 pour être introduit dans une artère 5.

La pompe PMP est configurée pour pomper le sang pauvre en oxygène et riche en dioxyde de carbone à un débit prédéfini et possiblement paramétrable. Le débit doit permettre une oxygénation et une décarboxylation efficace du sang pauvre en oxygène et riche en dioxyde de carbone.

La pompe PMP est également configurée pour que le sang injecté dans la canule d'injection 1 arrive sensiblement à la valeur d'un débit physiologique selon l'état du patient.

Selon un mode de réalisation, le système ECMO comprend un réservoir (non représenté). Ce dernier a pour fonction de réguler le débit de sang traité. La canule d'admission 2 prélève un volume F0 de sang qui est acheminé jusqu'au réservoir. Selon un mode de réalisation, cette étape est réalisée avant l'étape de pompage.

Selon un exemple de réalisation, la pompe PMP est de type « centrifuge ». Une pompe PMP centrifuge utilise le mouvement de rotation d'une roue insérée dans la pompe PMP. Ainsi, le déplacement et le débit du fluide peuvent être fixés et contrôlés. Selon un autre mode de réalisation, la pompe PMP est « à galets » également appelée « péristaltique ». Selon un mode de réalisation, la pompe PMP génère un débit pulsé.

L'oxygénateur OXY comporte une membrane qui reproduit artificiellement la fonction de la membrane alvéolo-capillaire. Cette membrane permet de réaliser des échanges gazeux afin d'oxygéner le sang et d'éliminer le dioxyde de carbone contenu dans le sang par décarboxylation. L'oxygénateur OXY est relié à la pompe PMP à partir de laquelle il reçoit le sang pauvre en oxygène et riche en dioxyde de carbone à un débit fixé.

Selon un mode de réalisation, la membrane de l'oxygénateur OXY est plane ou tubulaire. Les oxygénateurs OXY à membranes planes comportent des membranes en silicone ou des membranes assemblées en couches. Les membranes tubulaires sont composées de fibres creuses composées, par exemple de polyméthylpentènes non poreuses.

Selon un mode de réalisation, les fibres peuvent comprendre un revêtement offrant moins de résistance à l'écoulement et favorisant un écoulement de flux laminaire.

L'échangeur de chaleur ECH reçoit le sang oxygéné et décarboxylé de l'oxygénateur OXY pour le réchauffer. En effet, le sang passe par un système permettant de transférer l'énergie thermique d'un fluide tel que l'eau vers le sang, sans mélanger les deux fluides. Le flux thermique traverse la surface d'échange qui sépare le sang oxygéné et l'eau. L'échangeur thermique ECH fixe la température du sang afin qu'elle soit comprise dans l'intervalle de température du sang circulant dans le corps d'un patient.

Selon les modes de réalisation, l'échangeur peut être intégré au dispositif ECMO ou être externe à ce dernier.

Selon un mode de réalisation l'échangeur de chaleur ECH est relié à un réservoir de sorte à réchauffer le sang après son drainage, les étapes de pompage puis d'oxygénation/décarboxylation sont alors réalisées par la suite.

De manière alternative, selon un autre mode de réalisation, le chauffage peut provenir de l'oxygénateur lui-même. Dans ce cas, il comprend avantageusement des résistances prévues à cet effet.

L'orifice de l'extrémité proximale de la canule d'injection 1 est relié à l'échangeur thermique ECH de sorte qu'il reçoive le sang oxygéné, décarboxylé, et chauffé. Le sang oxygéné, décarboxylé, et chauffé entre dans une lumière principale LP de la canule d'injection 1. Une fraction du volume de sang injecté est ensuite capté par une lumière accessoire LAc agencée à l'intérieur de la lumière principale LP afin d'être injectée dans l'artère 5. Les deux lumières sont agencées de sorte que :
- le flux capté par la lumière accessoire LAc est injecté dans un sens antérograde selon un flux F2 dans l'artère 5 et ;
- le flux non capté par la lumière accessoire LAc et poursuivant dans la lumière principale LP est injecté dans un sens rétrograde selon un flux F1 dans l'artère 5.

Le flux sanguin dans l'artère 5 est représenté par les flèches « S » sur la figure 1.

La canule d'admission 2, la pompe PMP, l'oxygénateur OXY, l'échangeur thermique ECH et la canule d'injection 1 sont reliés entre eux par l'intermédiaire de quatre interfaces qui peuvent prendre la forme de tubes T1, T2, T3 et T4. Ces tubes T1, T2, T3, T4 sont destinés à faire passer le sang oxygéné ou non, décarboxylé ou non, entre les différents éléments permettant son traitement.

Ainsi, le tube T1 permet de relier l'orifice de la canule d'admission 2 à la pompe PMP. Le tube T2 permet de relier la pompe PMP à l'oxygénateur OXY. Le tube T3 permet de relier l'oxygénateur OXY à l'échangeur thermique ECH et le tube T4 permet de relier l'échangeur thermique ECH à l'orifice de la canule d'injection 1.

Le diamètre de chaque tube T1, T2, T3, T4 est choisi de sorte à permettre au sang de se déplacer tout en minimisant les risques d'hémolyse et de coagulation. Les diamètres des tubes T1, T2, T3, T4 sont donc ajustés selon les débits connus permettant la circulation du sang dans le corps.

Selon un mode de réalisation, les tubes T1, T2, T3, T4 ont un revêtement en héparine, un anticoagulant qui permet ainsi d'éviter la formation de caillots de sang à l'intérieur des tubes T1, T2, T3, T4.

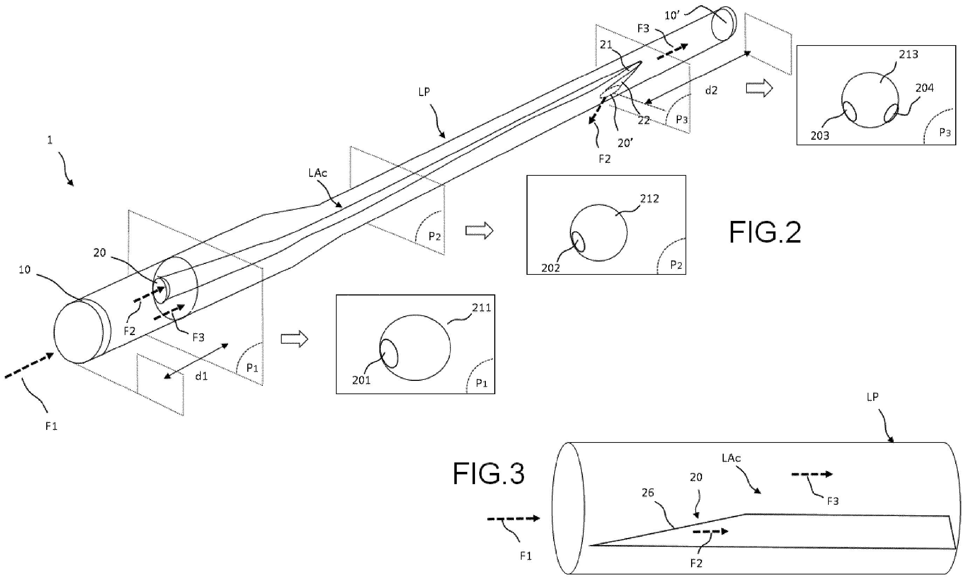

La figure 2 représente une vue en perspective d'une canule de l'invention 1. Selon un usage préféré correspondant à un fonctionnement avec une ECMO-VA périphérique, la canule 1 de l'invention est une canule d'injection. Selon un autre mode de réalisation, la canule de l'invention pourrait être utilisée comme une canule d'admission en utilisant un ou deux points de ponction pour prélever un volume de sang. Dans la suite de la description, le mode de réalisation d'une canule d'injection est décrit.

La canule d'injection 1 comprend une lumière principale LP comportant une extrémité proximale 10 apte à coopérer avec une embouchure de liaison provenant d'un composant de l'ECMO comme par exemple l'échangeur de chaleur ECH.

Une seconde lumière, dite lumière accessoire LA, est agencée à l'intérieur de la lumière principale LP. Selon un mode de réalisation, la lumière accessoire LAc comporte une portion qui s'étend parallèlement à la lumière principale LP. En outre, la lumière accessoire LAc comporte une portion coudée 21 débouchant sur une ouverture latérale 20' de la lumière principale LP.

### Agencement des deux lumières

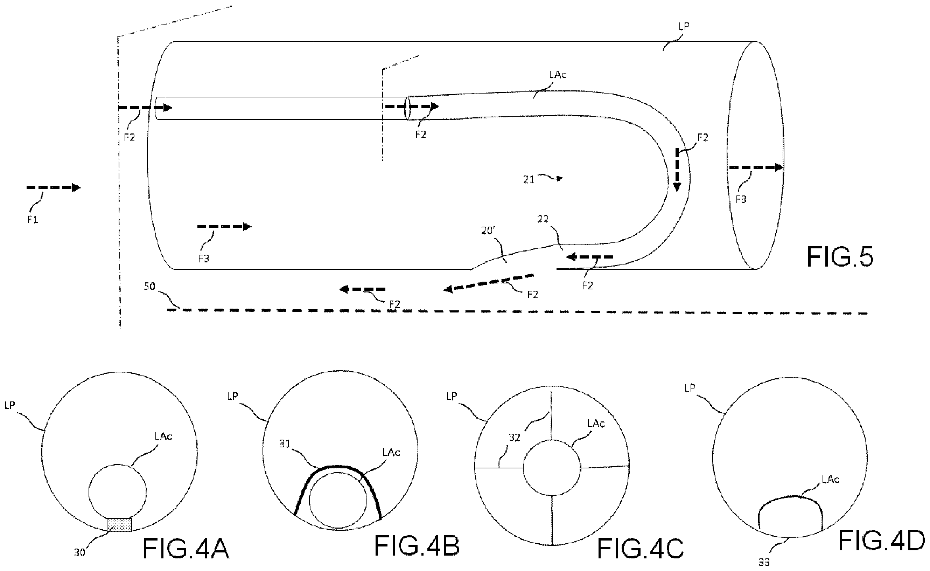

Selon un premier mode de réalisation, la lumière accessoire LAc est une lumière structurelle dissociée de la lumière principale LP. Les figures 4A à 4C représentent différentes variantes de réalisation dans chacune desquelles une coupe de chacune des lumières LAc et LP est représentée. Dans ce cas de figure, la lumière accessoire LAc peut être fixée à la lumière principale LP par une fixation souple ou rigide 30, 31, 32. Selon un mode de réalisation, la canule 1 comprend des moyens de maintien 30, 31, 32 de la lumière accessoire LAc. Par exemple, les moyens de maintien peuvent être des anneaux de fixation 31 répartis le long d'une portion de la lumière principale LP. Selon un autre exemple de réalisation, les moyens de maintien peuvent être formés d'un rail 30 s'étendant le long d'une portion des deux lumières, la lumière accessoire LAc et la lumière principale LP comprenant alors respectivement un connecteur longitudinal male et respectivement un connecteur longitudinal femelle ou inversement coopérant ensemble de sorte à solidariser les deux lumières LAc et LP ensembles.

Selon une autre variante de réalisation, la lumière accessoire LAc est maintenue sensiblement au centre de la lumière principale LP. Des moyens de maintien 32 forment des tiges semi-rigides ou rigides de sorte à maintenir en position la lumière accessoire LA.

Selon un second mode de réalisation, la lumière accessoire LAc est fixée sur une partie de la surface intérieure de la lumière principale LP qui s'étend selon la direction de la lumière LP. Dans ce mode de réalisation, la canule 1 comporte, par exemple, une double lumière LAc, LP qui peut être fabriquée par moulage. La figure 4D représente un exemple de réalisation dans lequel la lumière accessoire LAc et la lumière principale LP partagent une portion commune 33 de leur surface. Selon un autre mode de réalisation, la lumière accessoire LAc peut-être assemblée par collage à l'intérieur de la lumière principale LP.

Selon un mode de réalisation, la lumière accessoire LAc comporte un diamètre compris entre 20G et 6F. Lorsque la lumière accessoire LAc comporte des rétrécissements ou des élargissements de section, son plus grand diamètre ou son diamètre moyen est également compris entre 20G à 6F. Lorsque la canule accessoire LAc a un profil autre qu'un profil de section circulaire, comme par exemple elliptique, une dimension caractéristique équivalente au diamètre peut caractériser la canule. Cette dimension caractéristique est alors comprise dans une dimension équivalente à un diamètre de 20G et 6F.

L'extrémité proximale 20 de la lumière accessoire LAc est préférentiellement disposée en aval de l'extrémité proximale 10 de la lumière principale LP de manière à ce que le volume de liquide F2 entrant dans la lumière accessoire LAc corresponde à une fraction d'un volume F1 s'écoulant en amont de cette extrémité.

Dans la présente description, les notations F1, F2 et F3 représentent indifféremment un volume de liquide ou un débit de liquide selon le contexte dans lequel l'annotation est employée.

Selon un mode de réalisation, l'extrémité proximale 20 de la lumière accessoire LAc est disposée à quelques centimètres de l'extrémité proximale 10 de la lumière principale LP, par exemple cette distance d1 peut être comprise entre 5 et 10cm.

La lumière accessoire LAc comprend une portion 21 formant un coude permettant de diriger le flux F2 de liquide prélevé du flux F1 dans une direction sensiblement opposée à la direction du flux du liquide F3 non prélevé par la lumière accessoire LAc et poursuivant sa course dans la lumière principale LP vers l'extrémité distale 10'. La portion coudée 21 est agencée à l'intérieur de la lumière principale LP et débouche sur une ouverture latérale 20' de la lumière principale LP et donc de la canule 1 qui correspond à l'extrémité distale 20' de la lumière accessoire LAc.

Cet agencement évite qu'une partie saillante ne dépasse de la lumière principale LP. Cela est particulièrement souhaitable lors de l'introduction et du retrait de la canule 1 dans une artère 5 afin d'éviter tout endommagement de l'artère 5 avec une partie dépassant de la paroi de la canule 1.

L'invention a donc, grâce à la partie coudée de la lumière accessoire LAc, le double avantage de :
- permettre de délivrer un flux sanguin F2 dans le sens antérograde lors de l'introduction de la canule 1 dans une artère 5 et ;
- éviter d'endommager l'artère lors de l'introduction ou le retrait de la lumière principale LP.

En outre, l'invention évite la réalisation d'un second point de ponction d'une canule de reperfusion branchée en Y sur la ligne artérielle. Elle facilite donc la mise en place d'une ECMO-VA périphérique en n'ayant qu'un unique point de ponction de la canule 1 à réaliser.

La partie coudée 21 est représentée à la figure 2. Cette partie coudée 21 peut, selon les modes de réalisation, être plus ou moins étirée pour favoriser la création d'un flux laminaire et pour éviter les effets de turbulences engendrées par le coude 21.

Selon un mode de réalisation, la courbure du coude 21 est constante sur la portion coudée de sorte à minimiser les effets de la variation de courbure sur le fluide. Dans ce dernier cas de figure, la portion coudée 21 forme un arc de cercle, voire un demi-cercle pour modifier l'orientation du fluide F2 dans une direction antérograde, c'est-à-dire dans une direction opposée à l'éjection du fluide F3 en sortie 10' de la lumière principale LP.

L'ouverture 20' est adaptée au diamètre ou à la dimension caractéristique de la lumière accessoire LAc. La vitesse d'éjection du fluide F2 peut donc être contrôlée en fonction du débit entrant F1 dans la canule 1.

La figure 2 représente également trois plans de coupes P₁, P₂ et P₃. Un premier plan de coupe P₁ représente une section 201 de lumière accessoire LAc et une section de 211 de la lumière principale LP. Les deux lumières LAc et LP partagent une même portion de paroi comme cela est représenté à la figure 4D.

Un second plan de coupe P₂ représente une section 202 de lumière accessoire LAc et une section de 212 de la lumière principale LP. Les deux lumières LAc et LP partagent une même portion de paroi. On note que dans ce plan de coupe, les diamètres de lumières LAc et LP se sont réduits de manière à offrir une canule 1 occupant le minimum de place une fois introduite dans une artère 5. La réduction des diamètres permet également d'accélérer les débits injectés, en conséquence de diminuer l'effort hydrodynamique à fournir par l'ECMO.

Le rétrécissement des deux canules LAc et LP et le rapport des sections des deux canules LAc et LP permettent un contrôle de la vitesse d'éjection du sang dans l'artère 5 dans les deux directions. Le débit entrant F1 peut donc être calibré de sorte que les débits F3 et F2 correspondent à des débits physiologiques ou des débits physiologiques compensés selon si le fluide entre dans un sens antérograde ou rétrograde.

Un troisième plan de coupe P₃ est représenté dans la portion coudée 21 de la lumière accessoire LAc. On comprend alors que les sections 203 et 204 de la lumière accessoire LAc forment des voies acheminant le volume de fluide F2 dans des directions opposées. La section de la lumière principale LP est notée 213 sur la figure 2.

Selon un mode de réalisation, la lumière principale LP peut comprendre un bouchon partiel agencé sur une portion distale de la canule 1 permettant de compenser l'absence de la lumière accessoire LAc en amont afin de maintenir une même vitesse de fluide en sortie de la canule 1. Selon un autre mode de réalisation, la lumière principale LP ne comporte pas de bouchon partiel et la vitesse d'éjection ralentie vis-à-vis de la vitesse d'écoulement du fluide dans la lumière principale LP en présence de la lumière accessoire LAc est dimensionnée ainsi.

La distance d2 sur la figure 2 représente la distance entre l'orifice de la lumière accessoire LAc et l'extrémité distale de la lumière principale LP. La portion distale dans laquelle il n'y a plus de lumière accessoire a une longueur d2 moins la longueur du coude qui peut selon les modes de réalisation de la canule de l'invention varier selon le modèle de la canule de l'invention. Le modèle de la canule peut notamment varier selon mode opératoire prévu.

L'orifice de sortie 20' peut être avantageusement disposé selon les modèles de canules 1 à différentes distances de l'extrémité 10' de la lumière principales LP. Selon le gabarit des patients, le choix de l'artère à ponctionner et la position du point de ponction sur ladite artère de la canule 1, une canule appropriée est choisie selon les critères suivants :
- le diamètre d'extrémité distale 10' de la lumière principale LP ;
- la distance d2 entre l'orifice distal 20' de la lumière accessoire LAc et l'extrémité distale 10' de la lumière principale LP prédéfinie.

Selon un autre mode de réalisation, la longueur de la lumière accessoire LAc est beaucoup plus courte que la longueur de la lumière principale LP et correspond à une fraction de 5% à 50% de sa longueur. Selon un autre exemple de réalisation, la lumière accessoire peut être un unique coude 21 comportant quelques centimètres, voire quelques millimètres, en amont captant une fraction F2 du liquide circulant dans la lumière principale LP et quelques centimètres, voire quelques millimètres, en retour sur la portion 22 pour réorienter le flux F2 de liquide capté vers l'orifice 20'. Selon un mode de réalisation, l'orifice distal 20' de la lumière accessoire LAc et l'extrémité 10' de la lumière principale LP sont espacés d'une distance inférieure à 10 cm, voire inférieure à 5 cm.

Selon un mode de réalisation, la longueur du retour du bras coudé 22 à l'intérieur de la lumière principale LP peut être compris entre quelques millimètre et quelques centimètres. Selon un mode de réalisation, la longueur du retour du bras coudé 22 est comprise entre 1cm et 5cm. Cette longueur permet notamment de diminuer les turbulences et faire déboucher un liquide en sortie 20' laminaire.

Selon un mode de réalisation, la lumière accessoire LAc comporte une entrée biseautée 26 à son extrémité proximale 20 qui permet de :
- rendre laminaire le flux de liquide entrant dans la section de la lumière accessoire LA et/ou ;
- diminuer les perturbations du flux ou encore et/ou ;
- stabiliser le débit du flux de reperfusion du membre et/ou,
- limiter l'hémolyse.

Selon un mode de réalisation, la lumière accessoire LAc comporte une section qui forme un cercle ou une ellipse.

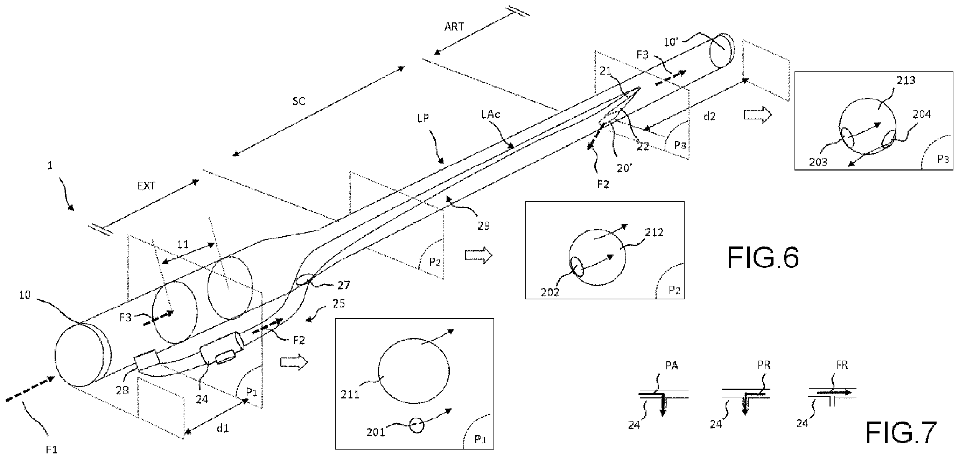

Selon une variante de réalisation, une portion externe 25 de la lumière accessoire LAc est agencée extérieurement à la lumière principale LP. La figure 6 représente ce mode de réalisation dans lequel la portion externe 25 comprend une entrée 20 agencée sur la surface latérale de la lumière principale LP et une sortie 27 agencée en aval de l'entrée 20 sur la surface latérale de la lumière principale LP. Selon ce mode de réalisation, la lumière accessoire LAc comporte une partie interne 29 agencée à l'intérieur de la lumière principale LP. Le mode de réalisation de la portion interne 29 est semblable aux différents modes de réalisation décrits précédemment au regard des figures 2 et des figures 4A à 4D. L'entrée 28, également appelée jonction, de la lumière accessoire LAc qui est déportée en amont de la canule 1 du fait de la présence de la portion externe 25 de la lumière accessoire LAc présente une différence avec le mode de réalisation de la figure 2.

Selon un mode de réalisation, la portion externe 25 de la lumière accessoire LAc est munie d'un robinet 24 permettant d'effectuer des purges.

Sur la figure 7 sont représentés les différents modes de fonctionnement d'un tel robinet 24.

Selon un premier mode de fonctionnement, une première purge antérograde, notée PA, peut-être réalisée lorsque le flux F2 s'écoule selon le sens indiqué sur la figue 5 en allant de l'amont vers l'aval de la canule et que le robinet 24 est ouvert.

Selon un second mode de fonctionnement, une seconde purge rétrograde, notée PR, peut-être réalisée lorsque le flux F2 s'écoule selon le sens inverse que le sens indiqué sur la figue 5 en allant de l'aval vers l'amont de la canule et que le robinet 24 est ouvert. Cette purge peut survenir lorsqu'un flux sanguin de reflux est généré lors de l'introduction de la canule dans un vaisseau.

Selon un troisième mode de fonctionnement, le flux F2 de la lumière accessoire LAc est acheminé vers la sortie 20' lorsque le robinet 24 est fermé. Le flux F2 parcourt la portion externe 25 et est réintroduit à l'intérieur de la lumière principale LP après la jonction 27 qui permet à la lumière accessoire LAc d'être prolongée à l'intérieur de la lumière principale LP. Dans ce mode de réalisation, la portion amont EXT de la canule 1 est destinée à rester à l'extérieur du corps du patient. En fonctionnement, la portion SC de la canule 1 est sous cutanée et la partie ART correspond à la portion introduite dans un vaisseau comme par exemple une artère.

Un avantage de ce mode de réalisation est de pouvoir bénéficier d'une fonction de purge permettant de vérifier la bonne position intra-artérielle de l'orifice de sortie 20', tout en ayant une canule à double lumière permettant d'éviter la mise en place d'une canule de reperfusion. Dans ce mode de réalisation, la canule présente deux portions dont l'une comprend une lumière accessoire LAc externe à la lumière principale LP pour réaliser, le cas échéant, des purges et une autre portion dans laquelle la lumière accessoire LAc est à l'intérieur de la lumière principale pour éviter une reperfusion.

Le mode de réalisation de la figure 6 présente selon un mode de réalisation, les mêmes caractéristiques que le mode de réalisation de la figure 2 dont notamment : une terminaison en coude, des accès et des sorties biseautées et possiblement un mode de fabrication analogue.

Selon un mode de réalisation, l'entrée 28 est prolongée en amont par une portion de la lumière accessoire LAc à l'intérieur de la lumière principale afin de prélever plus facilement un volume de sang. A titre d'exemple, l'entrée de la lumière accessoire LAc peut présenter la même interface que celle de la figure 3 à l'intérieur de la lumière principale LP avant de former un coude vers l'entrée 28 de la figure 6.

Le plan de coupe P1 représente les deux sections 211, 201 d'une part de la lumière principale LP et d'autre part de la lumière accessoire LAc. Les deux portions sont disjointes du fait que la coupe est faite au niveau où la lumière accessoire Lac forme une portion externe 25 à la lumière principale LP.

Le plan de coupe P2 représente un plan de coupe identique à celui de la figure 2 puisque la lumière accessoire LAc est dans cette portion interne à la lumière principale LP.

Le plan de coupe P3 représente un plan de coupe identique à celui de la figure 2 puisque la lumière accessoire LAc est dans cette portion comprise à l'intérieur du volume de la lumière principale LP et forme un coude semblable à celui de la figure 2.

La figure 5 représente une autre vue d'un mode de réalisation de la canule 1 de l'invention dans laquelle la partie coudée 21 est agrandie. On comprend que le volume F2 de fluide débouche sur une sortie latérale 20' de la lumière principale LP. La direction du fluide F2 en sortie 20' de la lumière accessoire LAc est donc modifiée vis-à-vis de la direction du même fluide F2 en amont du coude. La déviation du flux F2 du fait de l'angle formé par le coude 21 vers d'orienter un débit sortant 2 en sortie de la lumière accessoire LAc dans une direction antérograde. En outre, la figure 5 représente la paroi 50 de l'artère 5. Cette représentation permet de comprendre qu'une fois éjecté le fluide F2 se dirige sous l'effet de sa cinétique dans l'artère 5 dans la direction opposée à l'éjection du fluide F3 à l'extrémité distale de la lumière principale LP. Le fluide est éjecté à une certaine vitesse de la canule accessoire LAc et est guidé :
- d'une part, par la surface externe de la canule 1 et ;
- d'autre part, par la paroi interne 50 de l'artère 5.

Selon un mode de réalisation, le matériau utilisé pour la fabrication des lumières de la canule d'injection 1 de l'invention est en PVC semi-rigide thermosensible.

Selon un mode de réalisation, la partie de la canule destinée à être insérée dans les tissus puis dans un vaisseau du patient est armée afin d'éviter les coudures et clampages intempestifs.

Un avantage de la canule 1 de l'invention est de disposer d'une unique canule associant la fonction de reperfusion artérielle antérograde du membre à la mise en place d'une canule d'injection artérielle rétrograde. La reperfusion systématique diminue significativement les complications de l'ECMO-VA périphérique.

Par ailleurs, la canule 1 de l'invention permet de mettre en place en une seule procédure d'injection artérielle rétrograde dans l'aorte et la reperfusion antérograde dans le membre. Cette procédure offre un gain de temps précieux en temps chirurgical, anesthésique et réanimatoire. Cette unique procédure permet d'effectuer une unique ponction artérielle afin de réaliser l'injection artérielle rétrograde dans l'aorte et la reperfusion antérograde dans le membre. La diminution du nombre de ponctions permet de diminuer les risques d'embols artériels distaux, de dissection artérielle, de thrombose artérielle, et d'hématome au point de ponction.

Selon une invention d'une canule non revendiquée, la canule est identique à l'un des modes de réalisation de la canule précédente sauf en ce que l'extrémité distale peut ne pas correspondre avec l'ouverture de la lumière principale LP mais comprend au moins la portion externe 25 de la lumière accessoire LAc agencée extérieurement à la lumière principale LP. Par exemple, l'extrémité distale de la lumière auxiliaire peut être une portion qui est mobile entre une position intérieure à l'intérieure de la lumière principale LP pour l'installation de la canule dans l'artère et une position externe à l'extérieure de la lumière principale lorsque celle-ci fonctionne.

Par exemple lors du retrait d'un introducteur, la lumière auxiliaire passe de la position intérieure à la position extérieure.

Bien entendu cette canule peut avoir un robinet de purge comme décrit pour la figure 6.

Dans cette canule ou la canule comprenant l'extrémité distale de la lumière accessoire correspondant à l'ouverture latérale de la paroi de de la lumière principale, la portion externe 25 de la lumière accessoire LAc agencée extérieurement à la lumière principale LP comprend au moins une partie laissant passer la lumière à l'intérieur de la portion externe.

Ainsi, lors de la mise en place de la canule, l'utilisateur peut voir si l'extrémité distale de la lumière accessoire est dans l'artère, du fait du sang remontant dans cette lumière et donc passant au niveau de la portion externe laissant passer la lumière. En effet cette portion externe est destinée à être une portion extracorporelle de la canule.

En effet, même si la lumière principale est transparente ou translucide, il peut être difficile de voir si du sang coule dans la portion de lumière accessoire située dans la lumière principale et encore plus difficile d'évaluer visuellement si le débit est correct ou s'il y a un problème de circulation du fluide.

Cette partie peut être transparente ou translucide. L'intérêt qu'elle soit translucide est que l'opérateur puisse déterminer visuellement le débit du fluide traversant cette lumière accessoire.

Selon une invention d'une canule non revendiquée, la canule est identique à l'un des modes de réalisation des canules précédentes sauf en ce qu'elle peut avoir ou pas avoir : une portion externe 25 ou/et une extrémité distale correspondant avec l'ouverture de la lumière principale LP,
et en ce qu'elle comprend en outre une valve anti-reflux entre l'extrémité distale de la lumière principale et l'extrémité proximale de la lumière accessoire.

Cela permet de diminuer, voir supprimer, lors de l'installation de la canule, que du sang sorte de la canule par l'extrémité proximale de la lumière principale.

La valve anti-reflux n'est pas représentée sur les figures.

La valve anti-reflux peut avoir une ouverture ou une fente permettant d'y insérer un introducteur.

La valve anti-reflux peut avoir une ouverture ou une fente permettant d'y insérer un introducteur. L'introducteur traverse donc la valve anti reflux. La valve anti reflux comprend une membrane ayant l'ouverture ou la fente agencée de manière que les parois de la membrane ferme l'ouverture ou la fente lorsque du fluide pousse la membrane vers l'extrémité proximale de la lumière principale.

La membrane est agencée pour laisser le fluide circuler dans le sens de l'extrémité proximale de la lumière principale vers l'extrémité distale de la lumière principale.

Un autre objet non-revendiqué et ne formant donc pas partie de l'invention concerne aussi un ensemble canule introducteur comportant une des trois canules décrites précédemment et un introducteur ayant une portion traversant la lumière principale.

## Revendications

1. Canule (1) pour la circulation d'un fluide dans une artère (10), **caractérisée en ce qu'**elle comprend :
■ Une lumière principale (LP) acheminant un volume de fluide (F3) vers une première extrémité distale (10') ;
■ Une lumière accessoire (LA) comportant au moins une portion interne (29) agencée à l'intérieur de la lumière principale (LP) et s'étendant en partie parallèlement à cette dernière sur une portion de la longueur de la lumière principale (LP), ladite lumière accessoire (LA) comportant :
o une extrémité proximale (20) destinée à capter une fraction (F2) du débit entrant (F1) du fluide dans la lumière principale (LP) ;
o une portion coudée (21) modifiant la direction d'écoulement du débit de fluide captée (F2) par la lumière accessoire (LAc) vis-à-vis de la direction d'écoulement du fluide débouchant de la première extrémité (10') ;
o une seconde extrémité distale (20') située en amont de la première extrémité distale (10') de la lumière principale (LP), débouchant sur une ouverture latérale (23) de la canule (1) de sorte à diriger ladite fraction captée (F2) du liquide dans la direction modifiée d'écoulement (1), la seconde extrémité distale (20') correspondant à l'ouverture latérale (23).

2. Canule (1) selon la revendication 1, **caractérisée en ce que** la portion coudée oriente le flux (F2) capté par la lumière accessoire (LAc) dans une direction sensiblement opposée au flux s'écoulant (F3) vers l'extrémité de la lumière principale (LP).

3. Canule (1) selon l'une quelconque des revendications 1 à 2, **caractérisée en ce que** la lumière accessoire (LAc) est formée d'une unique portion interne (29).

4. Canule (1) selon l'une quelconque des revendications 1 à 2, **caractérisée en ce que** la lumière accessoire (LAc) comprend en outre une portion externe (25) à la lumière principale (LP) agencée en amont de la portion interne (29), la dite portion externe (25) présentant un robinet de purge (24) et étant destinée à être une portion extracorporelle (EXT).

5. Canule (1) selon la revendication 4, **caractérisée en ce que** la portion externe (25) de la lumière accessoire (LAc) comporte une première jonction (28) agencée au contact de la surface latérale de la lumière principale (LP) et une seconde jonction (27) agencée au contact de la surface latérale de la lumière principale (LP) de sorte à acheminer une fraction (F3) du liquide (F1) extérieurement à la lumière principale (LP) entre les deux jonctions.

6. Canule (1) selon l'une quelconque des revendications 1 à 5, **caractérisée en ce que** la lumière principale (LP) comporte un diamètre de 15F à 20F, soit de 5 à 7 mm et que la lumière accessoire (LAc) compote un diamètre de 20G à 6F, soit de 0,9 à 2mm.

7. Canule (1) selon l'une quelconque des revendications 1 à 6, **caractérisée en ce que** la lumière accessoire (LAc) a une section circulaire ou elliptique.

8. Canule (1) selon l'une quelconque des revendications 1 à 7, **caractérisée en ce que** l'extrémité proximale et/ou distale de la lumière accessoire (LAc) est biseautée de sorte que la section de l'embouchure proximale et/ou distale de la lumière accessoire (LAc) s'inscrive dans un plan non orthogonal à l'axe de l'une des deux lumières (LAc, LP).

9. Canule (1) selon l'une quelconque des revendications 1 à 8, **caractérisée en ce que** la lumière accessoire (LAc) comporte une partie longitudinale fixée le long d'une portion de la surface intérieure de la lumière principale (LP).

10. Système d'injection d'un fluide dans une artère, **caractérisé en ce qu'**il comporte :
■ Une canule (1) selon l'une quelconque des revendications 1 à 9,
■ une pompe (PMP) agencée pour assurer le pompage d'un débit de fluide prédéterminé (F0) provenant d'une canule de prélèvement (2), coopérant avec une entrée de la pompe via un tube d'interface (T1);
■ un oxygénateur (OXY) agencé pour introduire une proportion d'oxygène dans un fluide, une entrée de l'oxygénateur (OXY) coopérant avec une sortie de la pompe (PMP) via un second tube d'interface (T2) ;
■ un échangeur de chaleur (ECH) agencé pour injecter un fluide à une température fixée dans la canule (1) par l'intermédiaire d'un troisième tube d'interface (T4), ledit échangeur prélevant le fluide de l'oxygénateur (OXY) par l'intermédiaire d'un troisième tube (T3).

11. Système selon la revendication 10, **caractérisé en ce qu'**au moins un tube est recouvert d'une couche d'héparine.

12. Système selon l'une quelconque des revendications 10 à 11, **caractérisé en ce que** la pompe est agencée et configurée pour établir un débit de sang pulsé.

## Patentansprüche

1. Kanüle (1) für den Flüssigkeitsfluss in einer Arterie (10), **gekennzeichnet durch**:
• Einen Hauptlumen (LP), das ein Fluidvolumen (F3) zu einem ersten distalen Ende (10') führt;
• Ein Nebenlumen (LA), das zumindest einen internen Abschnitt (29) enthält, der innerhalb des Hauptlumens (LP) angeordnet ist und teilweise parallel zu diesem über einen Teil der Länge des Hauptlumens (LP) verläuft, wobei das Nebenlumen (LA) Folgendes umfasst:
o Ein proximales Ende (20), das dazu bestimmt ist, einen Anteil (F2) des einströmenden Fluidflusses (F1) im Hauptlumen (LP) aufzunehmen;
o Einen gebogene; Abschnitt (21), der die Flussrichtung des erfassten Fluidflusses (F2) durch das Nebenlumen (LAc) in Bezug auf die Flussrichtung der aus dem ersten distalen Ende (10') austretenden Flüssigkeit ändert;
o Ein zweites distales Ende (20'), das stromaufwärts des ersten distalen Endes (10') des Hauptlumens (LP) liegt und in eine seitliche Öffnung (23) der Kanüle (1) mündet, um besagten erfassten Anteil (F2) der Flüssigkeit in die geänderte Flussrichtung (1) zu leiten, wobei das zweite distale Ende (20') der seitlichen Öffnung (23) entspricht.

2. Kanüle (1) nach Anspruch 1, **gekennzeichnet dadurch, dass** der gebogene Abschnitt den erfassten Fluss (F2) durch das Nebenlumen (LAc) in eine im Wesentlichen entgegengesetzte Richtung zum fließenden Fluss (F3) in Richtung des Endes des Hauptlumens (LP) lenkt.

3. Kanüle (1) nach einem der Ansprüche 1 bis 2, **gekennzeichnet dadurch, dass** das Nebenlumen (LAc) aus einem einzigen internen Abschnitt (29) besteht.

4. Kanüle (1) nach einem der Ansprüche 1 bis 2, **gekennzeichnet dadurch, dass** das Nebenlumen (LAc) zusätzlich einen externen Abschnitt (25) zum Hauptlumen (LP) enthält, der stromaufwärts des internen Abschnitts (29) angeordnet ist, wobei besagter externe Abschnitt (25) ein Spülventil (24) aufweist und dazu bestimmt ist, ein extrakorporaler Abschnitt (EXT) zu sein.

5. Kanüle (1) nach Anspruch 4, **gekennzeichnet dadurch, dass** der externe Abschnitt (25) des Nebenlumens (LAc) eine erste Verbindung (28) umfasst, die an der seitlichen Oberfläche des Hauptlumens (LP) in Kontakt steht, und eine zweite Verbindung (27), die an der seitlichen Oberfläche des Hauptlumens (LP) in Kontakt steht, um einen Anteil (F3) der Flüssigkeit (F1) zwischen den beiden Verbindungen extern zum Hauptlumen (LP) zu leiten.

6. Kanüle (1) nach einem der Ansprüche 1 bis 5, **gekennzeichnet dadurch, dass** das Hauptlumen (LP) einen Durchmesser von 15F bis 20F, d.h. von 5 bis 7 mm, und das Nebenlumen (LAc) einen Durchmesser von 20G bis 6F, d.h. von 0,9 bis 2 mm, aufweist.

7. Kanüle (1) nach einem der Ansprüche 1 bis 6, **gekennzeichnet dadurch, dass** das Nebenlumen (LAc) einen kreisförmigen oder elliptischen Querschnitt aufweist.

8. Kanüle (1) nach einem der Ansprüche 1 bis 7, **gekennzeichnet dadurch, dass** das proximale und/oder distale Ende des Nebenlumens (LAc) abgeschrägt ist, sodass der Querschnitt der proximalen und/oder distalen Öffnung des Nebenlumens (LAc) in einer Ebene liegt, die nicht orthogonal zur Achse eines der beiden Lumina (LAc, LP) ist.

9. Kanüle (1) nach einem der Ansprüche 1 bis 8, **gekennzeichnet dadurch, dass** das Nebenlumen (LAc) einen länglichen Teil aufweist, der entlang eines Abschnitts der inneren Oberfläche des Hauptlumens (LP) befestigt ist.

10. Flüssiginjektionssystem in einer Arterie, **gekennzeichnet durch**:
• Eine Kanüle (1) nach einem der Ansprüche 1 bis 9,
• Eine Pumpe (PMP), die angeordnet ist, um einen vorgegebenen Flüssigkeitsfluss (F0) von einer Entnahmekanüle (2) zu fördern, und die mit einem Pumpeneinlass über ein Schnittstellenrohr (T1) zusammenwirkt;
• Ein Oxygenator (OXY), der angeordnet ist, um einen Sauerstoffanteil in eine Flüssigkeit einzuführen, wobei ein Einlass des Oxygenators (OXY) mit einem Pumpenauslass (PMP) über ein zweites Schnittstellenrohr (T2) zusammenwirkt;
• Ein Wärmeaustauscher (ECH), der angeordnet ist, um eine Flüssigkeit mit einer festgelegten Temperatur über ein drittes Schnittstellenrohr (T4) in die Kanüle (1) einzuspritzen, wobei der Austauscher die Flüssigkeit aus dem Oxygenator (OXY) über ein drittes Rohr (T3) entnimmt.

11. System nach Anspruch 10, **gekennzeichnet dadurch, dass** mindestens ein Rohr mit einer Heparin-Schicht überzogen ist.

12. System nach einem der Ansprüche 10 bis 11, **gekennzeichnet dadurch, dass** die Pumpe angeordnet und konfiguriert ist, um einen pulsierenden Blutfluss zu erzeugen

## Claims

1. A cannula (1) for fluid flow in an artery (10), **characterized in that** it comprises:
• A main lumen (LP) conveying a volume of fluid (F3) to a first distal end (10').
• An accessory lumen (LA) comprising at least one internal portion (29) arranged inside the main lumen (LP) and extending partly parallel to it over a portion of the length of the main lumen (LP), said accessory lumen (LA) comprising:
• A proximal end (20) intended to capture a fraction (F2) of the incoming flow (F1) of the fluid in the main lumen (LP);
• A bent portion (21) modifying the flow direction of the captured fluid flow (F2) by the accessory lumen (LAc) in relation to the flow direction of the fluid exiting the first distal end (10');
• A second distal end (20') located upstream of the first distal end (10') of the main lumen (LP), opening into a side opening (23) of the cannula (1) to direct said captured fraction (F2) of the fluid in the modified flow direction (1), the second distal end (20') corresponding to the side opening (23).

2. Cannula (1) according to claim 1, **characterized in that** the bent portion directs the captured flow (F2) by the accessory lumen (LAc) in a substantially opposite direction to the flowing flow (F3) towards the end of the main lumen (LP).

3. Cannula (1) according to any one of claims 1 to 2, **characterized in that** the accessory lumen (LAc) is formed by a single internal portion (29).

4. Cannula (1) according to any one of claims 1 to 2, **characterized in that** the accessory lumen (LAc) further comprises an external portion (25) to the main lumen (LP) arranged upstream of the internal portion (29), said external portion (25) having a purge valve (24) and being intended to be an extracorporeal portion (EXT).

5. Cannula (1) according to claim 4, **characterized in that** the external portion (25) of the accessory lumen (LAc) comprises a first junction (28) arranged in contact with the lateral surface of the main lumen (LP) and a second junction (27) arranged in contact with the lateral surface of the main lumen (LP) so as to convey a fraction (F3) of the fluid (F1) externally to the main lumen (LP) between the two junctions.

6. Cannula (1) according to any one of claims 1 to 5, **characterized in that** the main lumen (LP) has a diameter of 15F to 20F, i.e., from 5 to 7 mm, and the accessory lumen (LAc) has a diameter of 20G to 6F, i.e., from 0.9 to 2 mm.

7. Cannula (1) according to any one of claims 1 to 6, **characterized in that** the accessory lumen (LAc) has a circular or elliptical cross-section.

8. Cannula (1) according to any one of claims 1 to 7, **characterized in that** the proximal and/or distal end of the accessory lumen (LAc) is beveled so that the section of the proximal and/or distal orifice of the accessory lumen (LAc) fits into a plane that is non-orthogonal to the axis of one of the two lumens (LAc, LP).

9. Cannula (1) according to any one of claims 1 to 8, **characterized in that** the accessory lumen (LAc) comprises a longitudinal part fixed along a portion of the inner surface of the main lumen (LP).

10. A fluid injection system in an artery, **characterized in that** it comprises:
• A cannula (1) according to any one of claims 1 to 9,
• A pump (PMP) arranged to provide pumping of a predetermined fluid flow rate (F0) from a sampling cannula (2), cooperating with a pump inlet via an interface tube (T1);
• An oxygenator (OXY) arranged to introduce a proportion of oxygen into a fluid, an oxygenator inlet (OXY) cooperating with a pump outlet (PMP) via a second interface tube (T2);
• A heat exchanger (ECH) arranged to inject a fluid at a fixed temperature into the cannula (1) via a third interface tube (T4), said exchanger drawing fluid from the oxygenator (OXY) via a third tube (T3).

11. System according to claim 10, **characterized in that** at least one tube is coated with a heparin layer.

12. System according to any one of claims 10 to 11, **characterized in that** the pump is arranged and configured to establish a pulsatile blood flow.
